Europäisches Patentamt

European Patent Office

Office européen des brevets

(11) Publication number: **0 451 752 A2**

# EUROPEAN PATENT APPLICATION

(21) Application number: 91105509.3

(22) Date of filing: 08.04.91

(51) Int. Cl.5: **G01N 7/00, G01N 33/44**

(30) Priority: **12.04.90 IT 2003390**

(43) Date of publication of application:
**16.10.91 Bulletin 91/42**

(84) Designated Contracting States:
**DE FR GB**

(71) Applicant: **AFROS S.P.A.**
**Via Galileo Ferraris 33**
**I-21042 Caronno Pertusella Varese(IT)**

(72) Inventor: **Zanella, Paolo**
**Via Pick Mangiagalli 15**
**I-20141 Milan(IT)**

(74) Representative: **Coloberti, Luigi**
**Via E. de Amicis No. 25**
**I-20123 Milano(IT)**

(54) **Method and apparatus for testing gas dispersed in a liquid.**

(57) A testing method and apparatus for measurement of quantities of gas dispersed in a liquid, in particular nucleation air in a polyurethane component contained in a pressurized tank (14). In accordance with the invention a sample of the liquid to be tested, is draw from the tank (14) and fed into a testing cylinder (10) at the same pressure of the tank (14); the liquid sample is than compressed to a predetermined pressure value in the testing cylinder (10) and the quantity of dispersed gas is determined an referred to the atmospheric pressure by means of a suitable calculation algorithm as a function of the difference in volume caused by the compression.

The present invention relates to a testing method and apparatus for measurement of quantities of gas dispersed in a pressurized liquid and particular to calculate the percentage of nucleation air in a polyurethane component to be used in mixing heads and the like.

Nucleation air in liquid components normally used in the preparation of polyurethane mixtures, e.g. polyol, is necessary to solve several molding problems. In addition, the air acts as a nucleus for initiation of the chemical reaction between the polyurethane components after they have been appropriately mixed together. Therefore, during of the polyurethane components and before mixing, there must be dispersed therein a certain amount of nucleation gas or air, keeping and controlling the percentage of dissolved gas at a predetermined value.

At present, calculation of the quantities of nucleation gas dispersed or dissolved in a pressurized polyurethane component under oversaturation conditions is performed using various empirical systems which do not ensure correct testing or measurement of the percentage of nucleation gas in the component.

A first known system calls for measurement of the quantity of gas dispersed in a liquid as a function of the density of said liquid measured by means of a suitable float whose position in the liquid determines the percentage content of dissolved air. This system is very inaccurate because it does not allow for the pressure at which the measurement is made and which in part affects the density of said liquid. A second system, known from a previous Italian patent no. 1,161,944 of the same applicant, calls for taking a certain quantity of liquid from a tank under pressure and expanding it in a testing cylinder while calculating the percentage of dissolved gas as a function of the pressure of the gas in the testing cylinder at the end of expansion step. Since the nucleation gas dissolved in a liquid polyurethane component is normally found under oversaturation conditions, said system is well suited for liquids having low percentages of gas kept at relatively low pressures. In addition the system is affected negatively by the presence of other liquids having different vapour tensions which might altar the tested datum.

There is another system which calls for taking a sample of the liquid and allowing it to expand at atmospheric pressure and measuring or testing the percentage of dissolved gas as a function of the level reached by the foam at the end of the expansion. This system is also inaccurate and has limits because, during expansion, gas is freed and dispersed. Therefore, the volume at the end does not include all the air or gas which was actually dissolved or contained in the liquid at the beginning.

The object of the present invention is to provide a testing method and apparatus for measurement of quantities of gas dispersed in a liquid and suitable for operation under pressurized conditions such as to obviate the shortcomings of the above mentioned known systems.

Another object of the present invention is to provide a method and apparatus as set forth above which would allow very accurate and repeatable measurements not affected by pressure variations of the liquid to be tested or by the presence therein of other liquid components having different vapour tensions and such as not to cause any alteration of the tested datum.

Another object of the present invention is to provide an apparatus for measurement of quantities of nucleation gas dissolved in a polyurethane component and which, in addition to providing a continuous indication of the percentage of dissolved gas, would allow an automatic control, operating when necessary to restore the nucleation gas value in the storage tank.

These and other characteristics of the testing method and apparatus according to the invention will be described below with reference to the annexed drawing.

As shown, the apparatus comprises a testing cylinder 10, also termed "blind cylinder", inside of which there moves freely a piston 11. One side 12a of said cylinder is connected through a line 13 to a pressurized tank 14 containing a liquid to be tested, e.g. a polyurethane component in which is dissolved under conditions of oversaturation a gas such as nucleation air for a polyurethane mixture. Since it is necessary to ensure the substitution of the liquid upon each measurement or testing operation, it is necessary that the line 13 be short enough so that the volume of the liquid remaining therein is comparatively small, e.g. 10-15% of the volume of the tested sample. The tank 14 is also connected to a source 16 of compressed air through a valve 17 which may be operated to feed air or gas under pressure into the tank 14 to restore the percentage of dissolved gas when necessary.

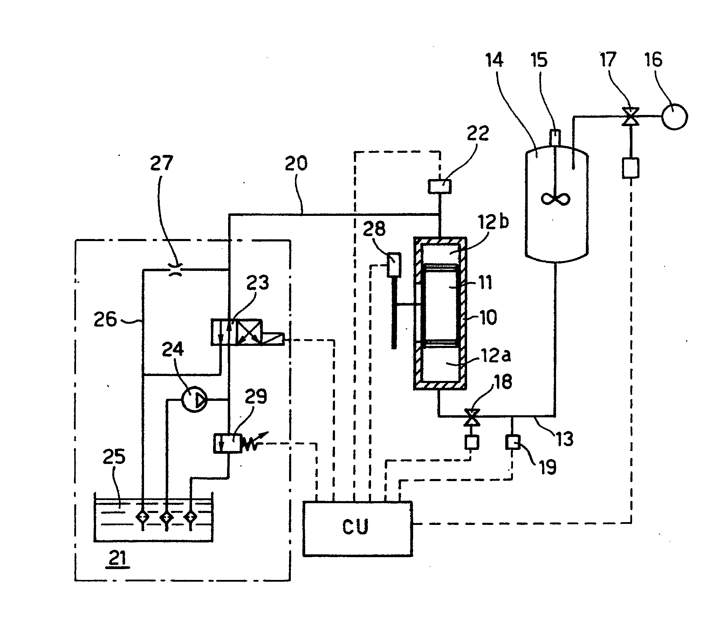

Reference number 18 in the drawing indicates a second automatically operated valve in the duct 13 connecting the cylinder 10 with the tank 14 to intercept or stop the passage of the fluid and allow compression of the liquid in the cylinder 10 during measurement. Reference number 19 indicates a pressure transducer for reading the pressure of the liquid in the tank 14. The other side 12b of the testing cylinder 10 is connected through a duct 20 to a hydraulic power unit 21 controlled by a control and calculation unit CU. In the example shown, 22 also indicates a second pressure switch on the line 20 designed to supply a signal indicating the compression value of the liquid sample in the chamber on the side 12a of the cylinder, given the proportionality between the pressures in the two chambers on the two sides 12a and 12b of the same cylinder 10.

More specifically, the chamber 12b of the testing cylinder is connectable through a distributor or solenoid valve 23 to the delivery side of a pump 24 or directly to the tank 25 of the power unit 21 containing a hydraulic fluid for operation of the cylinder 10. The line 20 supplying hydraulic fluid to the chamber 12b of the testing cylinder is also connected to the tank 25 through a branch duct 26 comprising a restricted orifice or passage 27. Reference number 28 indicates a linear positional sensing means operationally connected to the piston 11 of the testing cylinder, e.g. a potentiometer, designed to provide a signal of the position of the piston 11 which is proportional or equal to the volume which the chamber 12a progressively assumes as the piston moves.

Reference number 29 indicates a solenoid valve in a branch path for recycling the hydraulic fluid from the pump 24 to the tank 25.

All the control valves and the various sensing devices of the apparatus are connected to a central control and calculation unit CU which operates on the basis of a calculation algorithm defined below to control the entire operational cycle of the apparatus and indicate the percentage of nucleation air or gas in the liquid contained in the tank 14 with optional monitoring or printing of the datum supplied.

Operation of the apparatus described and method in accordance with the present invention are based on the principle of compressing a sample of liquid taken from the pressurized tank 14 or optionally from the feeding line to which it is connected, and reading the difference in volume caused by compression as compared with the known initial volume of the sample in the chamber 12a of the testing cylinder 10. This testing system is particularly well suited for measurement of high percentages of dissolved air in a liquid at relatively high pressures, e.g. 12 bars and higher.

The rodless cylinder 10 is therefore used to take a sample of the component from the tank 14 in a passive manner, i.e. allowing the same pressure of the liquid to cause retraction of the piston 11 and filling of the chamber 12a of the testing cylinder.

During filling of the chamber 12a the other side 12b of the cylinder is connected to the discharge pipe through the distributor 23 so that no significant counterpressure is exerted on the piston 11. It is important that filling of the chamber 12a of the testing cylinder take place freely, i.e. in a fully passive manner, allowing the piston 11 to retract freely in the cylinder 10 under the thrust of the pressure exerted by the liquid to be tested so as to avoid misrepresentation of the measurement, i.e. indication of the percentage of air in the sample as compared with that of the liquid in the tank 14. The volume of the sampled liquid is compared to a known volume of the chamber 12a, e.g. the volume which said chamber has at top dead centre of the piston 11, whose final position is read or sensed by the sensing device 28 which is capable of supplying a signal proportionate to the position of the piston 11 and hence the volumes of the liquid sampled after being taken at the pressure of the tank 14 and after compression respectively.

The valve 18, consisting for example of a ball valve operated by compressed air, serves to close the chamber 12a of the cylinder during compression while the pressure transducer 19 upstream of the valve 18 serves to supply the working pressure in the tank 14.

The algorithm for calculation of the percentage of undissolved air in the emulsion of the sampled liquid, normalized at atmospheric pressure, can be defined in the following manner. The data known or measured by means of the various transducers mentioned above are the following:

VC1 -  volume of the liquid sample in the cylinder 10 at the pressure in the tank 14,

VC2 -  volume of the liquid sample after compression,

PC1 -  pressure of the liquid in the tank 14 read by the pressure switch 19,

PC2 -  pressure of the sample in the cylinder 10 after compression. In general this pressure can be anything but there is a tendency to operate with high pressures.

The unknown value to be calculated is the percentage of nucleation air or gas contained in the emulsion of air and processing liquid. Since this value depends on pressure, the measurement is commonly related to the atmospheric pressure to obtain data which are always mutually comparable.

It is therefore desirable to calculate the percentage of air or gas in the sample at atmospheric pressure starting from the above data. In a first approximation the compressibility of the fluid in question is assumed to be null (an assumption which may be accepted as substantially correct because compared with the greater compressibility of the air or gas contained) and the compression undergone by the sample in the cylinder 10 is considered adiabatic given the relatively high speed of said compression, or polytropic with an index k very close to 1.4 (adiabatic K).

Starting from these assumptions it is possible to write the following equations 1 and 2.

1)    $VC1 = VF + VA1$
2)    $VC1 = VF + VA2 + \Delta VC$

where
    $VC1 =$    initial volume of the sample
    $VF =$    volume of the fluid in the sample
    $VA1 =$    volume of air in the sample at tank pressure
    $VA2 =$    volume of air in the sample after compression
    $\Delta VC =$    variation of the sample volume.

From equations 1 and 2 can be obtained the following equation no. 3.

3)    $VA1 = VA2 + \Delta VC$

where

$VA1$, $VA2$ and $\Delta VC$ have the meanings given above.

From the known equation of state for perfect gases

$$pV^k = \text{constant}$$

and on the basis of the above equation 3) it is possible to write the following equation 4).

4)    $PC1 \cdot VA1^k = PC2 \cdot VA2^k$

$k$, $VA1$ and $VA2$ have the meanings given above,
    $PC1 =$    tank pressure,
    $PC2 =$    pressure in the testing cylinder after compression.

Resolution of the system formed by the equations 3 and 4 (two equations in two unknowns) supplies the value of the air volume $VA1$ at tank pressure.

It is now possible to find the quantity of air dispersed in the sample related to the atmospheric pressure using the following relationship 5.

5)    $VA = VA1 \cdot PC1 / PA$

where
    $VA =$    percentage of air by volume in the sample related to the atmospheric pressure,
    $VA1 =$    the volume of air in the sample at tank pressure,
    $PC1 =$    tank pressure, and
    $PA =$    atmospheric pressure.

The percentage of air dispersed in the liquid is found by dividing $VA$ thus found by the initial sampled volume $VC1$ using formula 6.

6)    $VA\% = VA / VC1$

The working cycle of the apparatus can be repeated at predetermined time intervals, e.g. between approximately 20 and 30 seconds, and takes place in the following manner.

When at rest the chamber 12a of the cylinder 10 remains full of the component. Therefore at the beginning of the cycle the cylinder 10 is emptied by switching the chamber 12b on the drive side of the cylinder to discharge into the tank 25 by means of the distributor 23. At this point, by opening the valve 18 it is possible to perform passive filling of the chamber 12a of the cylinder 10 at the pressure of the tank 14, hence, after a few seconds of waiting, the pressure transducer 19 supplies said datum to the calculating unit CU.

Simultaneously, when the piston 11 reaches its top dead centre the transducer 28 supplies to the calculation unit CU an information relative to the volume of the sample in the cylinder. At this point the valve 18 between the cylinder 10 and the tank 14 closes upon a control signal provided by the unit CU, the hydraulic distributor 23 switches to connect the pump 24 to the drive side or chamber 12b of the testing cylinder 10 so that compression of the sample in the chamber 12a take place.

After reaching the desired pressure value, the signal supplied by the pressure switch 22 and the value of the volume of the compressed sample, which is proportional to the position signal given by the rheostat or transducer 28, are supplied to the CU unit.

At this point the control and calculation unit CU calculates the percentage of air in the sample at tank pressure and at atmospheric pressure using the above formulas and any monitoring and printout of the data taken.

Now the hydraulic distributor 23 is switched to allow total depressurization of the cylinder 10 by simultaneously opening the valve 18 connecting the cylinder 10 to the tank 14.

After opening of the valve 18 the hydraulic distributor 23 is switched and the piston 11 of the cylinder is operated to advance it to its lower dead centre, completely emptying the chamber 12a. The control unit 21 is again turned off and the hydraulic distributor 23 can be switched to begin another passive filling of the cylinder 10 and thus start another measurement cycle.

When the control and calculation unit CU finds a percentage of air in the sample lower than a predetermined value it commands the solenoid valve 17 to feed new air to the tank 14 and adjust it to the required percentage.

**Claims**

1.    A method for testing the quantity of gas dispersed in a pressurized liquid contained in a pressurized tank (14), in which a sample of liquid is fed to a testing cylinder (10) having a piston member (11), slidable therein, characterized by feeding said liquid sample to said testing cylinder (10), in a passive manner, at the same pressure of said tank (14) by allowing a freely movement of said piston member (11) slide in the testing cylinder (10) mentioned above; sensing the volume of said sample in said cylinder (10) at the pressure of said tank (14); compressing said sample in said cylinder (10) at a predetermined pressure value, sensing the volume of the sample at said compressed condition in the testing cylinder (10), and calculating the volume in percentage of gas dissolved in said liquid sample by difference of the volumes of the sample sensed in the testing cylinder (10) at the tank pressure and at said compressed condition respectively.

2. A method for measuring quantities of a gas dispersed in a liquid and particularly in a polyurethane component, according to claim 1, characterized by taking said liquid sample from said tank (14) in a passive manner by filling the cylinder (10) at the same pressure of said tank (14); compressing said liquid sample in said cylinder (10) until it reaches a predetermined pressure value; sensing the volumes of the sample in the cylinder (10) at tank pressure and at a compressed condition respectively; and by calculating the volume of gas in the sample at tank pressure through the difference in sensed volumes of the sample in the testing cylinder before and after compression, applying formula 1:

1) $VA1 = VA2 + \Delta VC$ where

$VA1 = $ volume of gas in the sample at tank pressure,

$VA2 = $ volume of gas in the sample after compression,

$\Delta VC = $ sample volume variation,

and then calculating the volume of gas in the sample related to atmospheric pressure using the equation of state for gas in accordance with formulas 2 and 3 below.

2) $PC1 \cdot VA1^k = PC2 \cdot VA2^k$

3) $VA = VA1 \cdot PC1 / PA$

where $VA1$ and $VA2$ have the meaning given above, and

$VA = $ air volume of the sample with reference to atmospheric pressure,

$PC1 = $ pressure of the sample before compression and corresponding to tank pressure,

$PC2 = $ pressure of the sample after compression.

3. A method in accordance with claim 2, characterized by calculating the percentage of gas dispersed in the liquid in the storage tank using the formula:-

$VA\% = VA / VC1$

where VA has the meaning referred above and VC1 is the initial volume of the liquid sample.

4. An apparatus for measuring quantities of gas dispersed in a pressurized liquid sample, characterized by comprising: a testing cylinder (10) having a freely slidable piston member (11), a side of said cylinder (10) being connected through a control valve (18) to a tank (14) containing the liquid to be tested; first control means (19) for providing a signal corresponding to the pressure of the liquid in the tank (14) and second control means (28) for providing informations of the volume of the liquid sample in the testing cylinder at tank pressure and after compression respectively; the other side of the testing cylinder (10) being operationally connected to a hydraulic power source (21), said control valve (18), said first control means (19), said second control means (28) and said hydraulic source (21) being operationally connected to a control unit (CU) for controlling and calculating the volume of the air dispersed in the liquid sample.

5. An apparatus in accordance with claim 4, characterized in that said first control means (19) for measuring the pressure in the tank consist of a pressure transducer positioned upstream of said control valve (18).

6. An apparatus in accordance with claim 4, characterized in that said second control means for providing the volumes of the liquid sample comprises a linear positional transducer (28) connected to the piston member (11) of said testing cylinder (10).

7. An apparatus in accordance with claim 6, characterized in that said transducer (28) consists of a potentiometric transducer.

8. An apparatus in accordance with claim 4, characterized by comprising a pressure transducer (19) designed to indicate the pressure of the sample in the testing cylinder (10), said pressure transducer (19)being operationally connected to said control and calculation unit (CU).